Europäisches Patentamt

European Patent Office

Office européen des brevets

(19)

(11) Publication number: **0 143 576**
A1

# EUROPEAN PATENT APPLICATION

(12)

(21) Application number: **84307818.9**

(22) Date of filing: **12.11.84**

(51) Int. Cl.⁴: **A 61 K 9/14,** A 61 K 47/00, A 61 K 33/10, C 01 F 7/00

(30) Priority: **18.11.83 CA 441465**

(43) Date of publication of application: **05.06.85 Bulletin 85/23**

(84) Designated Contracting States: **BE DE FR GB IT**

(71) Applicant: **WARNER-LAMBERT COMPANY, 201 Tabor Road, Morris Plains New Jersey 07950 (US)**

(72) Inventor: **Wright, Arthur P. G., 17 Presley Avenue, Scarborough Ontario, MLL 3P4 (CA)**
Inventor: **Ambike, Suhas H., 715 Military Trail, West Hill Ontario, M1E 4P6 (CA)**
Inventor: **Wen, Betty P., 24 Pepperell Crescent, Markham Ontario L3R 3G8 (CA)**
Inventor: **McKinnon, Michael A., 271 Albany Avenue, Toronto Ontario, M5R 3C7 (CA)**
Inventor: **Scheurer, Heinrich P., 57 Patterson Avenue, Scarborough Ontario MLL 3YL (CA)**
Inventor: **Blaser, Eric H., 36 Austin Terrace, Toronto Ontario M5R LY5 (CA)**

(74) Representative: **Jones, Michael Raymond et al, HASELTINE LAKE & CO. Hazlitt House 28 Southampton Buildings Chancery Lane, London WC2A 1AT (GB)**

(54) An antacid composition and a process for the preparation thereof.

(57) Fast-reacting dry antacids are formed by spray-drying fast reacting aluminum hydroxycarbonate gel with a heat stabilizer with or without the addition of magnesium hydroxide or carbonate added before or after spray-drying. Dry products containing magnesium and aluminum can be achieved having in vivo intragastric buffering efficacy superior to that of comparable liquid antacid suspensions.

EP 0 143 576 A1

ACTORUM AG

-1-

AN ANTACID COMPOSITION AND A PROCESS FOR THE
PREPARATION THEREOF

Aluminum hydroxide in wet gel form, i.e. in the form of a liquid
suspension, has many ideal antacid properties. However,
conventional wet gels contain only relatively low solids contents
of active antacid, and typically contain 80 to 90% water. The
transportation and storage of the wet gels are thus somewhat
inefficient and expensive. Further, the wet gels are not
particularly well adapted to the preparation of very concentrated
liquid antacids. Solid forms of antacid are not subject to these
disadvantages. Further, solid forms of antacid, e.g. in tablet
form, are very popular with consumers because they are much more
convenient and may be carried in small containers and taken as
needed without requiring a spoon or other measure for measuring
out the dosage.

Proposals have been made for the preparation of solid antacid
compositions by spray-drying aqueous slurries of aluminum
hydroxide gel containing heat stabilizers. For example, U.S.
Patent 3,272,704 dated September 13, 1966 in the name S.M.
Beekman discloses co-spray-drying mixed aqueous gels of aluminum
hydroxide or aluminum hydroxycarbonate and magnesium hydroxide,

0143576

magnesium carbonate or magnesium trisilicate, together with various heat stabilizers which stabilize the aluminum-containing gel and reduce the extent to which the antacid activity of the gel is impaired by the drying process.

Nevertheless, the use of antacid preparations in solid or tablet form has been considered to be subject to numerous disadvantages and limitations. Since tablets require mastication in the mouth they may not break up into the fine particle size exhibited by the conventional wet gel liquid suspensions which will easily reach the gastric areas affected by hyperacidity. Solid or tablet preparations which may exhibit good theoretical antacid reactivity, as determined by standard in vitro testing procedures, may not exhibit desired antacid reactivties in actual in vivo conditions of use.

Typically, antacids in solid or tablet form are slower to give relief from symptoms of hyperacidity because the antacid reactivity originally possessed by the aqueous gel-form antacid is much impaired by the step of drying it to the solid form.

Recently, methods have been developed for the preparation of aluminum hydroxycarbonate gels by continuous processing. The gels thus obtained exhibit fast acid-consuming reactivity even after the gel products are thoroughly washed to remove non-aluminum cations. The continuous processing reactions and the products obtained thereby are described in more detail in Canadian Patent No. 1,154,932 and in European Patent Application No. 83306293.8. It has now been found that when fast reacting aluminium hydroxycarbonate gel materials, having low molar ratios as typified, for example, by the products obtained from the above mentioned continuous processing methods, are co-spray-dried with a heat stabilizer, dry antacid compositions can be obtained exhibiting surprisingly fast acid-consuming reactivities. The dried product may be used as such as an antacid or its acid-consuming reactivity may be utilised in forming desired dry, solid aluminum and magnesium combining antacid compositions. Thus, the spray-dried aluminum hydroxycarbonate gel product may be blended with basic magnesium antacid materials, or the magnesium antacid material may be admixed with the wet hydroxycarbonate gel and the mixture may be co-dried from the slurry state to form aluminum-magnesium antacid preparations. Most unexpectedly, it has been found dry products can thus be obtained having in vivo intragastric buffering effects which are superior to those of conventional liquid suspension antacid preparations administered in dosages of comparable acid-consuming power. Further, the dried materials have the advantages that they are much easier and much less expensive to transport, and may be resuspended and rehydrated on admixture with water and flavour under high shear stirring conditions to yield smooth, highly concentrated liquid antacids.

In one aspect, the present invention provides an antacid composition comprising a co-spray-dried mixture of aluminum hydroxycarbonate and a water-soluble, orally ingestible heat stabilizer therefor, containing about 35 to about 450 parts by weight of said stabilizer per 100 parts of the hydroxycarbonate dry basis (calculated as $Al_2O_3$), and up to about 20% moisture by weight, and wherein said composition has a molar ratio of non-aluminum cations to aluminum cations of less than about 0.01:1, and its standard pH-stat reactivity times $T_{50}$ less than about 25 minutes and $T_{90}$ less than about 70 minutes.

As noted above, desirably, the dried product of the invention is characterized by certain standard pH-stat reactivity times. These reactivity times, as used throughout the present specification and claims, refer to the reactivity times determined by the pH-stat reactivity test described by Kerkhof et al, J. Pharm. Sci., Vol. 66, 1528 (1977). In this test, 1N HCl is added to the antacid composition undergoing test at a controlled rate such that a constant pH of 3.0 is maintained during the reaction. Unless otherwise specified, the test results referred to herein are obtained at a reaction temperature of 25°C. The volume of acid added is plotted against time. This plot can be characterized by $T_{50}$ and $T_{90}$ times, i.e. the times in minutes taken to consume respectively 50% and 90% of the total amount of acid added until neutralization. The volume of acid added can also be converted to the percentage total acid consuming power (% TACP)

$$\% \text{ TACP} = \frac{\text{Vol. acid consumed in titration}}{\text{Theoretical maximum volume}} \times 100$$

In the case in which the antacid is aluminum hydroxycarbonate, for example, the % TACP is given by

$$\% \text{ TACP} = \frac{\text{ml of 1N HCl} \times 102 \times 10^4}{\text{mg of hydroxycarbonate} \times \% \, Al_2O_3 \times 6}$$

Where 102 = Molecular weight of $Al_2O_3$

6 = Theoretical number of moles of acid consumed per mole $Al_2O_3$

The standard pH stat reactivity $T_{50}$ and $T_{90}$ times thus provide a significant indication of the reactivity of the antacid material. In the course of the spray-drying operation, even in the presence of the heat stabilizer, some loss of reactivity does of course take place. As noted above, in the spray-dried products in accordance with the invention, the $T_{50}$ and $T_{90}$ reactivity times should be less than about 25 minutes and less than about 70 minutes, respectively. More desirably, the $T_{50}$ and $T_{90}$ times are less than about 20 minutes and less than about 65 minutes, respectively. In order to achieve the desired relatively short reactivity times in the dried product, the starting material hydroxycarbonate wet gel should desirably possess prior to drying standard pH-stat reactivity times of $T_{50}$ less than about 20 minutes, and $T_{90}$ each less than about 25 minutes, more preferably with $T_{50}$ and $T_{90}$ each less than about 10 minutes. As the acid-consuming reactivities of the aluminum hydroxycarbonate gel materials tend to decline on prolonged storage, desirably the aluminum hydroxycarbonate gel material employed is one freshly obtained from a gel precipitation method.

As noted above, the product should also contain relatively low contents of non-aluminum cations. The presence of significant quantities of sodium or like cations is undesirable for dietetic reasons, and, further, products containing relatively low contents of non-aluminum cations may be desirable in order to meet requirements for maximum contents of non-aluminum cations prescribed for certain products. Thus, desirably, the starting material wet aluminum hydroxycarbonate gel material used as the starting material in the preparation of the present products has a molar ratio of non-aluminum cations to aluminum cations of less than about 0.01:1, more preferably less than about 0.005:1. Aluminum hydroxycarbonate gels, having fast reactivities and low contents of non-aluminum cations can be made, for example, by the continuous processing methods described in the above-mentioned Canadian patent 1,154,932 and co-pending European Patent Application No. 83306293.8, but the products of the present invention are not essentially limited to products derived from the hydroxycarbonate gel materials obtained from these processes, but can be achieved using starting material hydroxycarbonate wet gels of comparably fast reactivity and low non-aluminum cation content.

The preparation of the aluminum hydroxycarbonate gel material by the above-noted continuous processing methods is, however, attended by various advantages such as relatively high rates of throughput and small size of apparatus used for the manufacturing procedure, permitting significant economies to be achieved in the manufacturing process. Further, the continuous processing methods permit the gel product to be formed under highly homogeneous conditions, leading to a relatively high content of carbonate ion in the product which is permanently bound to the aluminum hydroxide molecular structure, thus permitting the gels to be well washed to significantly reduce their content of non-aluminum cations without concomitant signficant loss of reactivity of the gels. It is known that the acid-consuming reactivity of the gel is related to the presence of carbonate ion in the gel product. Typically, the well-washed hydroxycarbonate gel products obtained from the continuous processing methods will have molar ratios of $CO_3^{2-}$ ion to $Al^{3+}$ ion of from about 0.4 to about 0.8:1, more typically from about 0.45 to about 0.7:1. Because of the numerous advantages attendant on these materials, it is, therefore, highly preferred to employ the hydroxycarbonate gel materials obtained from the above-noted continuous processing methods as the starting material used in the preparation of the present spray-dried product.

The above described spray-dried aluminum hydroxycarbonate material can advantageously be used as such as an antacid. More advantageously, the material can be used in the preparation of dry powder or semi-dry pulverizable magnesium-aluminum antacid compositions by blending the spray-dried hydroxycarbonate product with magnesium carbonate and/or magnesium hydroxide in paste or powder form using conventional mixing apparatus. Any commercially available form of magnesium carbonate or magnesium hydroxide powder or water-containing paste which is in a state of purity rendering it acceptable for oral ingestion may be employed. More preferably, however, the magnesium compound in paste or powder form is added to the wet hydroxycarbonate gel, together with the heat stabilizer, and the wet mixture is co-spray-dried to yield a dry magnesium and aluminum-containing product mixture. It has been found that the presence of the magnesium compound in the wet slurry employed in the spray-drying procedure tends to further stabilize the aluminum hydroxycarbonate material against loss of acid-consuming reactivity during the spray-drying procedure, thus leading to products which have somewhat greater acid-consuming reactivities than those of mixtures of comparable chemical constitution formed by mixing the magnesium compound with the spray-dried aluminum hydroxycarbonate material in the dry state. In order to achieve products having desired antacid properties and wherein the content of mildly laxative magnesium compound tends to counteract the constipating action of the aluminum-containing component of the preparation, desirably the quantity of the magnesium compound combined with the aluminum hydroxycarbonate material, whether before or after spray-drying is an amount yielding in the final product an atomic ratio of Mg:Al ranging from about 0.5 to about 2.8:1. The basic magnesium compounds tend to exhibit a somewhat faster initial acid-consuming reactivity than the aluminum hydroxycarbonate material, and thus the standard pH-stat reactivity times of the magnesium and aluminum-containing mixtures, particularly the $T_{50}$ times of the mixed compositions, and, to a lesser extent the $T_{90}$ times, are reduced as compared with corresponding compositions containing solely the aluminum hydroxycarbonate material. Thus, mixtures having $T_{50}$ times less than about 10 minutes and $T_{90}$ times less than about 15 minutes can readily be achieved.

Thus, in accordance with a further aspect of the invention, there is provided an antacid composition comprising a co-spray-dried mixture of a basic magnesium compound selected from the group consisting of magnesium carbonate, magnesium hydroxide, and mixtures thereof, aluminum hydroxycarbonate and a water-soluble, orally ingestible heat stabilizer for the hydroxycarbonate and containing about 35 to about 450 parts by weight of said stabilizer per 100 parts of the hydroxycarbonate dry basis

(calculated as $Al_2O_3$), and up to about 20% by weight moisture, and containing said magnesium compound in an amount yielding an atomic ratio Mg:Al of from about 0.5 to about 2.8:1, the composition having a molar ratio of non-magnesium and non-aluminum cations to aluminum cations of less than about 0.01:1, and its standard pH-stat reactivity times $T_{50}$ less than about 10 minutes and $T_{90}$ less than about 15 minutes.

Preferably, the contents of magnesium compound in the mixture will be such as to yield an atomic ratio Mg:Al of from about 0.7 to about 2:1, still more preferably from about 0.8 to about 1.6:1.

Typically, the standard pH-stat reactivity times of the mixed products formed by co-spray-drying a mixture of the magnesium compound, stabilizer, and the hydroxycarbonate gel material from the wet state, will be $T_{50}$ less than about 5 minutes and $T_{90}$ less than about 10 minutes.

By reason of its ready availability, wide acceptance as an antacid material, and excellent acid-consuming properties, preferably the magnesium compound employed is magnesium hydroxide. Preferably, the magnesium compound, whether added before or after spray-drying is employed in the form of the commercially available aqueous-based pastes, as these tend to give products with better taste than equivalent products made using the magnesium compound in powder form.

The heat stabilizer incorporated in the wet slurry of aluminum hydroxide gel or mixed slurry of magnesium compound and aluminum hydroxycarbonate gel prior to spray-drying should be water-soluble, and should be of a standard of purity and in itself non-toxic and non-irritating to the gastric tract and free from toxic or irritating impurities, such that it is acceptable for oral ingestion. Various suitable materials are known which tend to stabilize aluminum hydroxide and hydroxycarbonate gel materials against impairment of their acid-consuming reactivities during hot drying. Frequently, these materials are water-soluble polyhydroxy compounds or relatively low molecular weight water-soluble organic polymers which on drying from aqueous solution tend to yield a gummy film or residue. Without wishing to be bound by any theory, it appears that the stabilizer materials, which, when dissolved, form part of the continuous aqueous phase in which the individual hydroxycarbonate gel particles are dispersed, tend to enrobe or coat the individual gel particles during the spray drying operation, thus physically shielding them from the hot gases to which the slurry particles are exposed during the spray-drying process. Examples of suitable stabilizer materials include glycine, hexitols such as sorbitol and mannitol, orally ingestible di- or tri-hydroxy alcohols such as butylene glycol and glycerine, poly(vinylpyrrolidone), methylcellulose, and hydroxypropyl methylcellulose. Frequently, the stabilizer materials not only stabilize the antacid reactivity of the materials during the spray-drying process, but also tend to stabilize the antacid reactivity of the dried products over prolonged periods of storage. Many of these stabilizer materials are also suspending aids, which facilitate the re-constitution of the spray-dried material, permitting it to be reconstituted with water under high shear mixing to form concentrated liquid antacid suspensions. Further, many of these stabilizer materials are tabletting aids, which facilitate the compaction of the dry powder products direct into tablet form using conventional tabletting presses.

Especially preferred for the purposes of the present invention are the hexitols such as mannitol and sorbitol, by reason of their excellent stabilizing properties, and by reason of their efficacy as re-suspension and tabletting aids. Because of its wide availability in a state of orally ingestible purity and its wide acceptance as an excipient and relatively low cost, sorbitol is particularly preferred. In order to achieve adequate stabilization of the hydroxycarbonate gel material against impairment of its reactivity during tne spray-drying operation a certain minimum weight ratio of the heat stabilizer to the hydroxycarbonate should be present. It is convenient for this purpose to calculate the amount of hydroxycarbonate present in

terms of its $Al_2O_3$ content. The amount of the stabilizer present in the slurry prior to spray-drying should be at least about 35 parts by weight per 100 parts of the hydroxycarbonate, dry basis (calculated as $Al_2O_3$). The use of excessive quantities of the stabilizer in the slurry is to be avoided, because the use of amounts above a certain level does not appear to contribute increased stabilizing effect and merely results in increased consumption of the stabilizer and can result in products which have undesirably low concentrations of active antacid material. Desirably, the amount of stabilizer should be no more than about 450 parts by weight per 100 parts of hydroxycarbonate dry basis (calculated as $Al_2O_3$). More preferably, the content of the stabilizer is about 80 to about 280 parts by weight, and still more preferably about 110 to 190 parts by weight per 100 parts of the hydroxycarbonate, dry basis (calculated as $Al_2O_3$).

In the spray-drying operation, which can be conducted using conventional spray-drying apparatus, it is desired to reduce the moisture content of the slurry, which typically will initially be about 70 to about 85%, to no more than about 20%, more preferably about 5 to about 15%, and still more preferably to about 10% by weight, in order to yield a free flowing powder product which is dry to the touch and which can readily be handled and transported and can be re-constituted to form concentrated liquid suspensions or can be pressed into tablets, with the addition if necessary of conventional re-suspending or tabletting aids. In order to avoid excessive impairment of the pH-stat reactivity times and percent TACP of the product, it is desirable to expose the material in the spray-drying operation to drying gases of relatively low temperature. Desirably, the drying is conducted employing dryer gas which at no point in the spray-drying tower or other spray-drying apparatus has a temperature in excess of about 400°C, more preferably no more than about 300°C.

Examples

## Example 1

A well washed wet aluminum hydroxycarbonate gel was obtained, prepared by the continuous processing method described in co-pending European Patent Application No. 83306293.8. The wet gel had a content of the hydroxycarbonate corresponding to 10.56% $Al_2O_3$ by weight, an Na:Al molar ratio of 0.003:1, a $CO_3^{2-}:Al^{3+}$ ratio of 0.517:1, standard pH-stat reactivity times $T_{50}$ = 6.3 minutes, $T_{90}$ = 8.7 minutes, and % TACP = 97.9. The wet gel material was mixed with sorbitol solution USP in an amount yielding in the mixture a content of 62% by weight of the sorbitol in the composition, dry basis, or approximately 220 parts by weight of the sorbitol per 100 parts of the hydroxycarbonate, dry basis (calculated as $Al_2O_3$). The sorbitol dissolved in the aqueous phase.

The resulting mixture was fed as a slurry to a conventional spray-drying apparatus employing a two fluid atomization nozzle and equipped with a cyclone for receiving the spray-dried product. The rate of feed of the slurry was about 18 kg/hr, the hot air supplied to the dryer had an inlet air temperature of about 343°C, an outlet air temperature of about 107°C. The spray-dried powder product recovered had a moisture content of about 10%.

Analysis of the spray-dried product showed an $Al_2O_3$ content of 28.12% by weight, an acid-consuming power of 15.62 meq/g, % TACP of 94.4, and standard pH-stat reactivity times $T_{50}$ of 17.8 and $T_{90}$ of 58.7 minutes.

The product could be employed as an antacid or could be dry-blended with dry magnesium hydroxide powder USP to yield a magnesium-aluminum antacid powder having excellent acid-consuming reactivity.

## Example 2

An aluminum hydroxycarbonate gel slurry was obtained similar to that described in example 1. A blend of 22.83 Kg of this gel plus 15 Kg of a 30% W/W Mg (OH)$_2$ paste USP, and 5.61 Kg of a 70% W/W Sorbitol USP solution was made. To this mixture, 20 Kg of water was added to make the blend pumpable and 30 ml of 5% hypochlorite bleach was added to prevent bacterial contamination of the slurry yielding a mixture containing a weight ratio Mg(OH)$_2$:aluminum hydroxycarbonate (measured as Al$_2$O$_3$) of 1.87:1 (corresponding to an Mg:Al atomic ratio of about 1.63:1), and having a sorbitol content of about 163 parts by weight per 100 parts aluminum hydroxycarbonate, dry basis (calculated as Al$_2$O$_3$). This mixture was then fed to the spray dryer at 21.5 Kg/hr with the inlet dryer temperature of 315°C and an outlet temperature of 107°C, yielding 12.7 Kg of powder. The dry product obtained had a moisture content of about 10%, an acid-consuming power of 22.47 meq/g, % TACP of 98.9 and standard pH-stat reactivity times $T_{50}$ = 4.0 minutes and $T_{90}$ = 19.7 minutes.

The dried product could be reconstituted with water and flavour under high-shear stirring to form a smooth, pleasant tasting, highly concentrated liquid antacid. Further, the dry composition could be pressed on conventional tabletting equipment to form antacid tablets.

## Example 3

An aluminum hydroxycarbonate gel slurry was obtained having a content of hydroxycarbonate (measured as Al$_2$O$_3$) 13.07 % by weight, an Na:Al molar ratio 0.0054, a $CO_3^{2-}$:Al$^{3+}$ molar ratio of 0.476, and having standard pH-stat reactivity times $T_{50}$ = 15.6 minutes, $T_{90}$ = 24.9 minutes and having % TACP = 99.8. The gel slurry was well mixed with sorbitol USP solution and with a commercially-available aqueous paste of magnesium hydroxide USP, yielding a mixture containing a weight ratio Mg(OH)$_2$:aluminum hydroxycarbonate (measured as Al$_2$O$_3$) of 1.02:1 (corresponding to an Mg:Al atomic ratio of about 0.89:1), and having a sorbitol content of about 130 parts by weight per 100 parts aluminum hydroxycarbonate, dry basis (calculated as $Al_2O_3$).

The slurry mixture was co-spray-dried under the drying conditions described above in Example 1. A dry product was obtained having a moisture content of about 10%, an acid-consuming power of 22.17 meq/g, % TACP of 99.7 and standard pH-stat reactivity times $T_{50}$ = 5.3 minutes and $T_{90}$ = 16.8 minutes.

The dried product could be reconstituted with water under high-shear stirring to form a smooth, pleasant tasting, highly concentrated liquid antacid. Further, the dry composition could be pressed on conventional tabletting equipment to form antacid tablets.

Example 4

An aluminum hydroxycarbonate gel slurry was obtained having a content of hydroxycarbonate (measured as $Al_2O_3$) of 7.12% by weight, a Na:Al molar ratio 0.0043:1, a $CO_3^{2-}:Al^{3+}$ molar ratio of 0.757, and having standard pH-stat reactivity times $T_{50}$ = 4.1 minutes, $T_{90}$ = 7.5 minutes and having % TACP = 100.5. The gel slurry was well mixed with sorbitol USP solution and with a commercially-available aqueous paste of magnesium hydroxide U.S.P., yielding a mixture containing a weight ratio $Mg(OH)_2$:aluminum hydroxycarbonate (measured as $Al_2O_3$) of 1.77:1 (corresponding to an Mg:Al atomic ratio of about 1.55:1), and having a sorbitol content of about 326 parts by weight per 100 parts aluminum hydroxycarbonate, dry basis (calculated as $Al_2O_3$).

The resulting slurry mixture was fed to a conventional spray-drying apparatus employing a rotary atomizer and equipped with a cyclone for receiving the spray-dried product. The rate of feed of the slurry was about 51 Kg/hr, the hot air supplied to the dryer had an inlet air temperature of about 290°C, an outlet air temperature of about 60 to 102°C, and was supplied at a rate of about 630 Kg/hr.

The spray-dried powder product was obtained having a moisture content about 8% and a sorbitol content of 50%. The magnesium:aluminum ratio was 1:1 (calculated as the weight ratio of $Mg(OH)_2:Al(OH)_3$). pH-stat reactivity tests on the dried composition using the standard pH-stat reactivity test described above, gave $T_{50}$ and $T_{90}$ times of 2.9 and 5.0 minutes respectively, an acid-consuming powder of 18.36 meq/g and a % TACP of 100.2%. The composition was pressed into tablets, each of which had an acid-neutralization capacity 13.25 meq. Each tablet contained a weight of aluminum hydroxycarbonate equivalent to 200 mg dried aluminum hydroxide gel and 200 mg of magnesium hydroxide. pH-stat reactivity tests on the tablets gave $T_{50}$ and $T_{90}$ times of 2.7 and 7.8 minutes, respectively and a % TANC of 96.7%.

Two of these tablets were given in the antacid clinical study for a total ANC of 26.5.

## Example 5

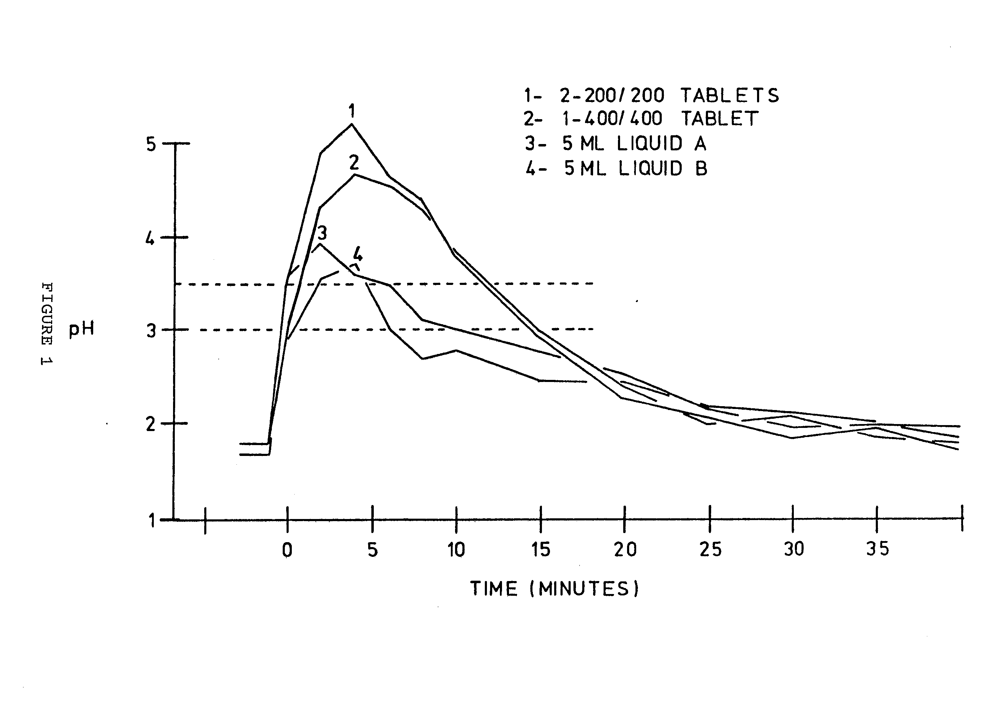

Figure 1 of the accompanying drawings shows a graph of intragastric pH against time following oral administration of various antacid compositions.

The procedure of Example 2 was followed to obtain a co-spray-dried antacid composition of moisture content about 10% and of which about 30% of the solids content of the composition was sorbitol. The magnesium/aluminum ratio was 1:1 (calculated as the weight ratio $Al(OH)_3:Mg(OH)_2$). pH-stat reactivity tests on the dried composition using the standard pH-stat reactivity test described above, gave $T_{50}$ and $T_{90}$ times of 4.0 and 19.7 minutes, respectively and a % TACP of 98.9. The composition was pressed into tablets, each of which had an acid neutralization capacity (ANC) determined by the USP XX test of 26.01 meq. Each tablet contained a weight of aluminum hydroxycarbonate equivalent to 400 mg $Al(OH)_3$, which contains 53.75% aluminum oxide, and 400 mg of $Mg(OH)_2$.

The intragastric buffering effect of the antacid tablets was compared with that of two commercially-available liquid magnesium-aluminum antacid suspensions. In vitro assay of liquid suspension A indicated pH-stat reactivity times of $T_{50}$ 19.4 minutes $T_{90}$ 30.8 minutes and % TANC of 99.3. Liquid suspension B gave pH-stat reactivity times of $T_{50}$ 26.9 minutes, $T_{90}$ more than 70 minutes, and % TANC of 90.5. Each of these liquid suspensions contained, per 5ml dose, 600 mg aluminum hydroxide equivalent to dried gel at 53.75% $Al_2O_3$ and 300 mg $Mg(OH)_2$.

Testing was performed with ten fasting normal adult subjects. In successive tests each subject received two tablets described in Example 4, one of the above-described tablets, one 5 ml dose of liquid suspension A, having an ANC of 28.4 meq/5 ml and a 5 ml dose of liquid suspension B, having an ANC of 26.8 meq/5 ml.

Before and during the course of the testing, intragastric pH readings were determined by measurement of the pH of aspirated samples of gastric fluid. The aspiration samples were obtained by means of a naso-gastric tube positioned in the antrum region of the stomach.

Baseline pH measurements were made before administraton of any tablet or liquid suspension at 5 minute intervals until three consecutive readings or pH 2.0 or less were obtained. After administration of the liquid suspension or tablet, pH was determined at 2 minute intervals up to 10 minutes after administration and at 5 minute intervals thereafter until the pH returned to baseline or 60 minutes time had elapsed. After each administration of the tablet or suspension, the tablet was chewed and swallowed or each 5 ml suspension was swallowed, and in each instance the administration was followed by 50 ml of distilled water.

The mean intragastric pH profiles obtained with the antacid tablet and with the two liquid antacid suspensions are shown in the graph of the accompanying drawing. As will be noted from the graph, the buffering parameters of the peak pH reached, the time the pH was elevated beyond pH 3.5 and of the duration of the buffering effect above the base line, above pH 3, and above pH 3.5 were all significantly greater with the antacid tablet than those obtained with the liquid suspensions A and B.

Thus the tablet formulation exhibited superior intragastric buffering efficacy as compared with the commercially-available liquid suspensions, particularly having regard to the fact that the ACP of the tablet (25.4 meq) was somewhat less than each 5 ml dose of liquid suspension A and liquid suspension B (28.4 and 26.8 meq respectively).

CLAIMS:

1. An antacid composition which comprises a co-spray-dried mixture of: (a) aluminium hydroxycarbonate; and (b) a water-soluble, orally ingestible heat stabilizer therefor in an amount of from 35 to 450 parts by weight per 100 parts by weight of the aluminium hydroxycarbonate, dry basis (calculated as $Al_2O_3$); the composition further containing 20% moisture by weight;

wherein the composition has a molar ratio of non-aluminium cations to aluminium cations of less than 0.01:1; and wherein its standard pH-stat reactivity times, $T_{50}$ and $T_{90}$ (as hereinbefore defined) are less than 25 minutes and less than 70 minutes respectively.

2. A composition according to claim 1, wherein the molar ratio of non-àluminium cations to aluminium cations is less than 0.005:1.

3. A composition according to Claim 1 or 2, wherein the reactivity times $T_{50}$ and $T_{90}$, are less than 20 minutes and less than 65 minutes respectively.

4. An antacid composition which comprises a co-spray-dried mixture of: (a) a basic magnesium compound chosen from magnesium carbonate, magnesium hydroxide, and mixtures thereof; (b) aluminium hydroxycarbonate; and (c) a water-soluble, orally ingestible heat stabilizer for the hydroxycarbonate in an amount of from 35 to 450 parts by weight per 100 parts by weight of the hydroxycarbonate, dry basis (calculated as $Al_2O_3$); the composition further containing up to about 20% moisture by weight;

wherein the magnesium compound is present in an amount yielding an atomic ratio Mg:Al of from 0.5 to 2.8:1, wherein the compoisition has a molar ratio of non-magnesium and non-aluminium cations to aluminium cations of less than 0.01:1, and wherein its standard pH-stat reactivity times $T_{50}$ and $T_{90}$, are less than 10 minutes and less than 20 minutes respectively.

-18-

5. A composition according to claim 4, wherein the atomic ratio Mg:Al is from 0.7 to 2:1, preferably from 0.8 to 1.6:1.

6. A composition according to claim 4 or 5, wherein the reactivity times, $T_{50}$ and $T_{90}$ are less than 5 minutes and less than 10 minutes respectively.

7. A composition according to Claim. 4, 5 or 6, wherein the magnesium compound is magnesium hydroxide.

8. A composition according to any preceding claim, containing from 80 to 280 parts by weight of the stabilizer per 100 parts of the hydroxycarbonate, dry basis (calculated as $Al_2O_3$), preferably from 110 to 190 parts by weight of the stabilizer per 100 parts of the hydroxycarbonate, dry basis (calculated as $Al_2O_3$).

9. A composition according to any preceding claim, wherein the stabilizer is chosen from one or more of: glycine, a hexitol, a di- or tri-hydroxy alcohol, poly-(vinylpyrrolidone), methylcellulose, and hydroxypropyl methylcellulose; preferably a hexitol, more preferably sorbitol.

10. A composition according to any preceding claim, wherein the moisture content is from 5 to 15%, preferably about 10%.

11. A process for the production of a dry antacid composition which process comprises: co-spray-drying, to a moisture content of up to 20% by weight, an aqueous slurry of aluminium hydroxycarbonate gel having dissolved, in the aqueous phase, an orally ingestible heat stabilizer for the hydroxycarbonate in an amount of about 35 to 450 parts by weight per 100 parts of the hydroxycarbonate, dry basis (calculated as $Al_2O_3$), wherein the hydroxycarbonate gel has, in the wet state, a molar ratio of non-aluminium cations to aluminium cationsof less than 0.01;1 and wherein its standard pH-stat reactivity times, $T_{50}$ and $T_{90}$ (as hereinbefore defined), are less than 20 minutes and less than 25 minutes respectively; and recovering a spray dried particulate product.

12. A process according to claim 11, further comprising blending the dry product with a basic magnesium compound in dry power or aqueous paste form, the magnesium compound being chosen from: magnesium carbonate, magnesium hydroxide and mixtures thereof, to yield an atomic ratio Mg:Al of from 0.5 to 2.8:1 and to form a product mixture.

13. A process for the production of a magnesium-containing dry antacid composition which process comprises: co-spray-drying, to a moisture content of up to 20% by weight, an aqueous slurry of aluminium hydroxycarbonate gel mixed with a basic magnesium compound chosen from magnesium carbonate, magnesium hydroxide and mixtures thereof in an amount to yield an atomic ratio Mg:Al of from 0.5 to 2.8:1, the aqueous slurry having dissolved, in the aqueous phase, an orally ingestible heat stabilizer for the hydro-carbonate in an amount of from 35 to 450 parts by weight per 100 parts of the hydroxycarbonate, dry basis (calculated as $Al_2O_3$), wherein the hydroxycarbonate gel has, in the wet state, a molar ratio of non-aluminium cations to aluminium cations of less than 0.01:1 and wherein its standard pH-stat reactivity times, $T_{50}$ and $T_{90}$, are less than 20 minutes and less than 25 minutes respectively; and recovering a spray-dried particulate product.

14. A process according to claim 12 or 13, wherein the atomic ratio Mg:Al is from 0.7 to 2:1, preferably 0.8 to 1.6:1.

15. A process according to claim 12, 13 or 14, wherein the magnesium compound is magnesium hydroxide.

16. A process according to any one of claims 11 to 15, wherein the hydroxycarbonate gel has, in the wet state, a molar ratio of $CO_3^{2-}$ ion to $Al^{3+}$ ion of from 0.4 to 0.8:1, preferably from 0.45 to 0.7:1.

17. A process according to any one of Claims 11 to 16, wherein the molar ratio of non-aluminium and

-20-

non-magnesium cations to aluminium cations is less than 0.005:1.

18. A process according to any one of claims 11 to 17, wherein the reactivity times, $T_{50}$ and $T_{90}$, are both less than 10 minutes.

19. A process according to any one of claims 11 to 18, wherein the slurry contains from 80 to 280 parts by weight of the stabilizer per 100 parts of the hydroxycarbonate, dry basis (calculated as $Al_2O_3$), preferably from 110 to about 190 parts by weight of the stabilizer per 100 parts of the hydroxycarbonate, dry basis (calculated as $Al_2O_3$).

20. A process according to any one of claims 11 to 19, wherein the stabilizer is chosen from one or more of: glycine, a hexitol, a di- or tri-hydroxy alcohol, poly(vinylpyrrolidone), methylcellulose, and hydroxypropyl methylcellulose, preferably a hexitol, more preferably sorbitol.

21. A process according to any one of claims 11 to 20, wherein the slurry is spray-dried to a moisture content of from 5 to 15%, preferably from 5 to 10%.

22. A process according to any one of claims 11 to 21, wherein the spray-drying is conducted in a dryer gas having an inlet temperature no more than 400°C.

23. A process according to any one of claims 11 to 21, therein the spray-drying is conducted in a dryer gas having an inlet temperature no more than about 300°C.

24. A process according to claim 16 or any one of claims 17 to 23 when appendent to claim 16, wherein the product has standard pH-stat reactivity times, $T_{50}$ and $T_{90}$, less than 25 mintues and less than 70 minutes respectively, preferably less than 20 minutes and less than 65 minutes respectively.

25. A process according to claim 12 or 13, or to any one of claims 14 to 23 when appendent to claim 12 or 13, wherein the particulate product or product mixture has standard pH-stat reactivity times, $T_{50}$ and $T_{90}$, less

than about 10 minutes and less than 15 minutes respectively.

26. A process according to any one of claims 11 to 25, including the step of compressing the particulate product or product mixture to form a tablet.

27. A process according to any one of claims 11 to 26, including the step of resuspending the particulate product or product mixture to form highly concentrated liquid antacids with an acid neutralization capacity of 45 to 55 milliequivalent per 5 ml.

FIGURE 1

European Patent
Office

**EUROPEAN SEARCH REPORT**

Application number

EP 84 30 7818

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-3 272 704 (S. BEEKMAN)<br><br>* Column 3, line 35 - column 4, line 23; column 13, line 50 - column 14, line 54 * | 1-27 | A 61 K 9/14<br>A 61 K 47/00<br>A 61 K 33/10<br>C 01 F 7/00 |
| X | GB-A-1 414 121 (W. SMITH)<br><br>* Page 4, lines 68-117 * | 1-27 | |
| X | CHEMICAL ABSTRACTS, vol.95, no.4, July 27, 1981, page 335,abstract no. 30344u, COLUMBUS, OHIO,(US). N. PRUCKKUMVONG et al.:"The effect of sorbitol on the reactivity of liquid antacids containing wet/ dried aluminum hydroxide gel".<br><br>& Varasarn Paesachasarthara 1980, 7(2), 29-40<br><br>* Abstract * | 1-27 | **TECHNICAL FIELDS SEARCHED (Int. Cl.4)**<br><br>A 61 K<br>C 01 F |
| A | US-A-2 999 790 (C. ALFORD)<br><br>* Column 3, line 21 - column 4, line 24 * | 1-27 | |

-/-

The present search report has been drawn up for all claims

| Place of search | Date of completion of the search | Examiner |
|---|---|---|
| THE HAGUE | 05-02-1985 | BRINKMANN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

**European Patent Office**

**·EUROPEAN SEARCH REPORT**

Application number

EP 84 30 7818

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl.4) |
|---|---|---|---|
| A | EP-A-0 088 958 (NIPPON SHINYAKU COMPANY LTD.)<br><br>* Page 12, claims 1-4 *<br><br>--- | 1-27 | |
| D,A | CA-A-1 154 932 (WARNER-LAMBERT LTD.)<br><br>--- | 1-27 | |
| A | JOURNAL OF PHARMACEUTICAL SCIENCES, vol.65, no. 8, August 1976, page 1195-8, WASHINGTON, DC, (US).<br>S. NAIL et al.:"Structure of Aluminum Hydroxide Gel III: Mechanism of Stabilization by Sorbitol".<br><br>* Page 1197 abstract *<br><br>----- | 1-27 | TECHNICAL FIELDS SEARCHED (Int. Cl.4) |

The present search report has been drawn up for all claims

| Place of search | Date of completion of the search | Examiner |
|---|---|---|
| THE HAGUE | 05-02-1985 | BRINKMANN |